(19) Europäisches Patentamt
European Patent Office
Office européen des brevets

(11) **EP 4 596 721 A1**

(12) **EUROPEAN PATENT APPLICATION**
published in accordance with Art. 153(4) EPC

(43) Date of publication:
**06.08.2025 Bulletin 2025/32**

(21) Application number: **23907058.4**

(22) Date of filing: **19.12.2023**

(51) International Patent Classification (IPC):
**C21B 5/06** (2006.01)  **C07C 1/04** (2006.01)
**C07C 1/12** (2006.01)  **C10K 3/06** (2006.01)
**C21B 5/00** (2006.01)  **C21B 13/02** (2006.01)
**F27D 17/00** (2025.01)  **C21B 13/00** (2006.01)

(52) Cooperative Patent Classification (CPC):
**C21B 13/0073; C07C 1/04; C07C 1/12; C10K 3/06;
C21B 13/02; F27D 17/00;** C21B 2100/22;
C21B 2100/26; C21B 2100/44; C21B 2100/64;
Y02P 10/143

(86) International application number:
**PCT/JP2023/045558**

(87) International publication number:
**WO 2024/135698 (27.06.2024 Gazette 2024/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.12.2022 JP 2022207628**

(71) Applicant: **JFE Steel Corporation**
**Tokyo 100-0011 (JP)**

(72) Inventors:
• NAKAHARA Yoshiko
 Tokyo 100-0011 (JP)
• TERUI Koki
 Tokyo 100-0011 (JP)
• NOUCHI Taihei
 Tokyo 100-0011 (JP)

(74) Representative: **Scott, Stephen John**
**YUJA IP LAW**
**4 Centenary House**
**The Avenue**
**York YO30 6AU (GB)**

(54) **REDUCED IRON MANUFACTURING METHOD**

(57) Provided is a method of producing reduced iron that can achieve both energy savings and a decrease in $CO_2$ emissions. A system is configured to circulate and reuse top gas and includes a blowing process, a reduction process, a distribution process, a synthesis process, and a reforming process. Additional $H_2O$ is supplied to the synthesis process, and an amount of the additional $H_2O$ supplied to the synthesis process is controlled according to amounts of CO and $H_2O$ contained in a first top gas.

FIG. 2

EP 4 596 721 A1

## Description

TECHNICAL FIELD

The present disclosure relates to a method of producing reduced iron.

BACKGROUND

In recent years, there has been a strong demand for energy savings in steelworks against the backdrop of global environmental issues and fossil fuel depletion issues. The raw material of iron is mainly iron oxide, and a reduction process to reduce this iron oxide is essential in steelworks. The most widespread and common reduction process worldwide uses a blast furnace. In a blast furnace, coke or pulverized coal reacts with oxygen in hot blast (air heated to about 1200 °C) from a tuyere. This reaction produces CO and $H_2$ as reducing gases, which are used to reduce the iron ore and the like in the furnace. Recent improvements in blast furnace operation technology have decreased the reducing agent rate (the amount of coke and pulverized coal used per tonne of hot metal produced) to about 500 kg/t, and the reducing agent rate has already almost reached a lower limit. Therefore, no further significant decrease in the reducing agent rate is expected.

On the other hand, in regions where natural gas is produced, a vertical reduction furnace (hereinafter also referred to as a shaft furnace) is often used to produce reduced iron. In this method, the reduction furnace is charged with sintered ore, pellets, or other agglomerated iron ore as the iron oxide raw material (hereinafter also simply referred to as iron oxide). Reducing gas including CO and $H_2$ is then blown into the reduction furnace to reduce the iron oxide to produce reduced iron. In this method, natural gas or the like is used as the feed gas for the reducing gas. This feed gas is heated and reformed together with top gas in a reformer. This produces reducing gas. Here, the top gas is gas after the iron oxide is reduced in the reduction furnace, and is typically discharged from the top of the reduction furnace. The reducing gas is blown into the reduction furnace and reacts with iron oxide supplied from the top of the furnace. The iron oxide is then reduced to reduced iron. The reduced iron is then cooled in a region below where the reducing gas is blown into the reduction furnace, and then discharged from the bottom of the reduction furnace.

Further, as mentioned above, the top gas, which is gas after iron oxide is reduced, is discharged from the reduction furnace, for example, from the top of the furnace. After dust collection and cooling is applied to the top gas, some is fed to the reformer as raw material for reformed gas. Further, remaining top gas is used as fuel gas for the reformer. In this method, the top gas used as fuel gas for the reformer is normally discharged out of the system.

As such a reduced iron production process, for example, Patent Literature (PTL) 1 describes a method of producing reduced iron by reforming exhaust gas from a reduction furnace and natural gas in a reformer to produce reducing gas consisting mainly of CO and $H_2$, blowing the reducing gas into the reduction furnace, and reducing iron oxide in the reduction furnace.

Further, PTL 2 describes a method of producing reduced iron by reforming coke oven gas and $CO_2$-removed top gas from a reduction furnace to produce reducing gas, which is then blown into a reduction furnace.

CITATION LIST

Patent Literature

   PTL 1: JP 2017-088912 A
   PTL 2: JP 6190522 B2

SUMMARY

(Technical Problem)

The method described in PTL 1 uses externally supplied natural gas for the production of reducing gas. Therefore, although lower than that of blast furnaces, there is a problem that a certain amount of $CO_2$ emissions are unavoidable.

Further, the method described in PTL 2 uses coke oven gas or converter gas produced in a steelworks to produce reducing gas. Here, in an integrated steelworks, coke oven gas and converter gas are needed as fuel gas for downstream processes such as a heating furnace and an annealing furnace. Therefore, when coke oven gas or converter gas is diverted to the reduced iron production process, a fuel gas shortage is caused in the downstream processes. As a result, natural gas is supplied from outside to compensate for the shortage of fuel gas in the downstream processes. That is, even with the method described in PTL 2, energy savings and a decrease in $CO_2$ emissions could not both be achieved, and the problem remained.

In view of the circumstances described, it would be helpful to provide a method of producing reduced iron that can realize both energy savings and a decrease in $CO_2$ emissions.

(Solution to Problem)

The inventors have conducted extensive research to realize both energy savings and a decrease in $CO_2$ emissions, and developed a system that circulates and reuses top gas.

That is, the inventors developed

a top gas circulation and reuse system (hereinafter also simply referred to a circulation system) comprising:

a blowing process of blowing reducing gas into a reduction furnace;
a reduction process, in the reduction furnace, of reducing iron oxide by the reducing gas to obtain reduced iron;
a distribution process of distributing top gas discharged from the reduction furnace into a first top gas and a second top gas;
a synthesis process of synthesizing regenerative methane gas from the first top gas and hydrogen gas; and
a reforming process of using the regenerative methane gas and the second top gas as feed gas to obtain the reducing gas from the feed gas.

The inventors further investigated and made the following discoveries.

- In order to stably carry out an operation that achieves both energy savings and a decrease in $CO_2$ emissions, it is important to maintain a healthy mass balance, in particular the mass balance of C (carbon), in the circulation system described above. In other words, it is important to control the gas balance of the circulation system so that the ratio of the amount of $H_2$ to the amount of CO in the reducing gas supplied to the reduction furnace (hereinafter also referred to as $H_2$/CO of the reducing gas) is always maintained within a certain range without significant fluctuations. Here, the $H_2$/CO of the reducing gas is the volume ratio under standard conditions (which may also be called the flow rate ratio under standard conditions). The same applies to $CO_2$/CO and the like, which is the ratio of the amount of $CO_2$ to the amount of CO contained in the top gas, as described below.
- The synthesis of $CH_4$ with high yield and stability in the synthesis process is necessary for this purpose.

Top gas normally contains a certain amount of CO that was not used for the reduction of iron oxide in the reduction furnace, along with $CO_2$. Therefore, in the synthesis process, $CH_4$ is synthesized from CO in addition to $CO_2$. When $CH_4$ is synthesized from $CO_2$, $CH_4$ is synthesized according to the methanation reaction equation in the following Expression (i). When $CH_4$ is synthesized from CO, $CH_4$ is synthesized according to the methanation reaction equation in the following Expression (ii).

$$CO_2 + 4H_2 \rightarrow CH_4 + 2H_2O \ \Delta H = \text{-165 kJ/mol ...} \qquad \text{(i)}$$

$$CO + 3H_2 \rightarrow CH_4 + H_2O \ \Delta H = \text{-206 kJ/mol ...} \qquad \text{(ii)}$$

However, when CO methanation reactions occur using typical methanation catalysts, catalyst poisoning occurs and the catalyst deactivates over a longer period of operation, resulting in lower methane yields.

Here, a huge pressure swing adsorption (PSA) separator or the like is needed to separate $CO_2$ from CO contained in the top gas. However, depending on facility specifications, it may not be possible to install such a huge apparatus. Further, even when such apparatus could be installed, installation is not necessarily desirable in terms of overall facility compactness, energy efficiency, and the like.

Therefore, the inventors further investigated a method of preventing catalyst poisoning in the synthesis process and stably synthesizing $CH_4$ at a high yield without separating the $CO_2$ and CO contained in the top gas in the circulation system described above.

As a result, the inventors made the following discovery.

Separately supplying $H_2O$ to the synthesis process, and controlling the amount of additional $H_2O$ supplied to the synthesis process according to the amount of CO and $H_2O$ contained in the first top gas. This enables stable synthesis of $CH_4$ at a high yield by preventing catalyst poisoning in the synthesis process, even without separating $CO_2$ and CO contained in the top gas. As a result, it is possible to operate with high operational stability and achieve both further energy savings and a decrease in $CO_2$ emissions in the production of reduced iron.

The inventors understand the above mechanism to work as follows.

That is, compounds with aldehyde (CHO) groups are formed as intermediates in the $CO_2$ methanation reaction. On the other hand, C is produced as an intermediate during the CO methanation reaction. This C precipitates on the catalyst and clogs the catalyst active sites, resulting in catalyst poisoning.

Here, when a sufficient amount of $H_2O$ is present in the synthesis process, C that is formed as intermediates during the CO methanation reaction and $H_2O$ react to form compounds with aldehyde (CHO) groups. This enables stable synthesis of $CH_4$ at a high yield by preventing catalyst poisoning in the synthesis process, even without separating $CO_2$ and CO contained in the top gas. As a result, it is possible to operate with high operational stability and achieve both further energy savings and a decrease in $CO_2$ emissions in the production of reduced iron.

The present disclosure is based on these discoveries and further studies.

Primary features of the present disclosure are as follows.

1. A method of producing reduced iron, the method comprising:

a charging process of charging iron oxide into a reduction furnace;
a blowing process of blowing reducing gas into the reduction furnace;
a reduction process, in the reduction furnace, of reducing the iron oxide by the reducing gas to obtain reduced iron;
a distribution process of distributing top gas discharged from the reduction furnace into a first top gas and a second top gas;
a synthesis process of synthesizing regenerative methane gas from the first top gas and hydrogen gas; and
an additional supply process of supplying additional $H_2O$ to the synthesis process; and
a reforming process of using the regenerative methane gas and the second top gas as feed gas to obtain the reducing gas from the feed gas, wherein
an amount of the additional $H_2O$ supplied to the synthesis process is controlled according to an amount of CO and an amount of $H_2O$ contained in the first top gas.

2. The method of producing reduced iron according to 1, above, wherein the amount of the additional $H_2O$ supplied to the synthesis process is controlled so that the sum of the amount of $H_2O$ contained in the first top gas and the amount of the additional $H_2O$ supplied to the synthesis process is from 1.0 equivalent to 3.0 equivalents relative to the amount of CO contained in the first top gas.

3. The method of producing reduced iron according to 1, above, wherein the amount of the additional $H_2O$ supplied to the synthesis process is controlled so that the sum of the amount of $H_2O$ contained in the first top gas and the amount of the additional $H_2O$ supplied to the synthesis process is from 1.0 equivalent to 2.0 equivalents relative to the amount of CO contained in the first top gas.

4. The method of producing reduced iron according to any one of 1 to 3, above, wherein $CO_2/CO$, a ratio of an amount of $CO_2$ to the amount of CO contained in the top gas, is from 0.25 to 4.0.

5. The method of producing reduced iron according to any one of 1 to 4, above, wherein de-dusting of the top gas or the first top gas is carried out prior to the synthesis process.

6. The method of producing reduced iron according to any one of 1 to 5, above, wherein dehydration of the regenerative methane gas is carried out prior to the reforming process.

(Advantageous Effect)

According to the present disclosure, it is possible to operate with high operational stability and achieve both further energy savings and a decrease in $CO_2$ emissions in the production of reduced iron.

BRIEF DESCRIPTION OF THE DRAWINGS

In the accompanying drawings:

FIG. 1 is a diagram illustrating a conventional reduced iron production process; and
FIG. 2 is a diagram illustrating an example of a reduced iron production process according to a method of producing reduced iron according to an embodiment of the present disclosure.

DETAILED DESCRIPTION

The following is a description of a method of producing reduced iron according to an embodiment of the present disclosure, with reference to the drawings.

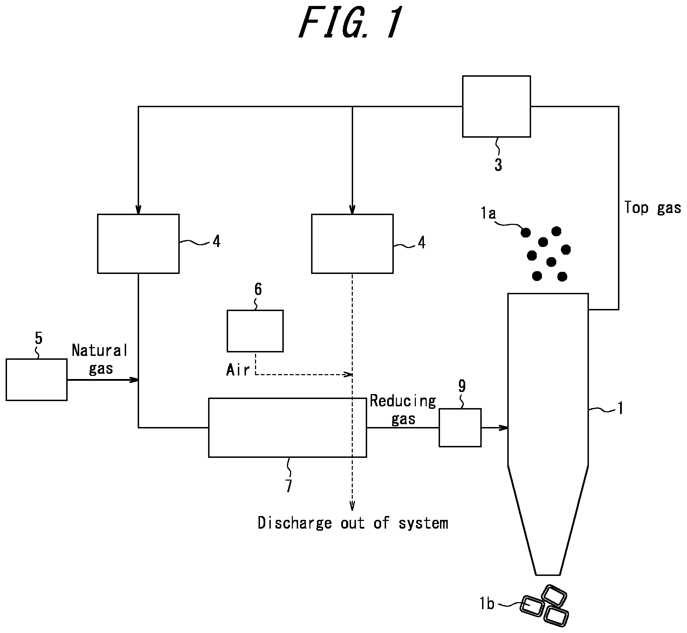

First, a conventional reduced iron production process (hereinafter also referred to as a conventional production process) is described. FIG. 1 is a schematic diagram illustrating an example of a conventional production process. In the drawing, reference sign 1 is a reduction furnace, 1a is iron oxide, 1b is reduced iron, 3 is a deduster, 4 is a dehydrator, 5 is a natural gas supply, 6 is an air supply, 7 is a reformer, and 9 is a reducing gas blowing device.

In the example of a conventional production process, iron oxide is charged from the top of the reduction furnace and gradually descends. There, iron oxide is reduced by blowing in high-temperature reducing gas from a central portion of the reduction furnace. The reduced iron is then discharged from the bottom of the reduction furnace. In this process, top gas containing mainly CO, $CO_2$, $H_2$, and $H_2O$ is discharged from the top of the reduction furnace. The top gas is de-dusted by the deduster, and a portion is subjected to moisture adjustment and fed to the reformer as feed gas. Gas containing hydrocarbons, for example, natural gas from a natural gas supply, is supplied to the reformer along with the top gas that has been subjected to moisture adjustment. The supplied gas is then heated in the reformer. A reforming reaction then occurs, producing high-temperature reducing gas containing mainly CO and $H_2$. This reducing gas is then blown into the reduction furnace. Further, the remaining portion of the top gas is dehydrated and used as fuel for heating in the combustion chamber of the reformer, for example, by combustion with oxygen in the air. After combustion as fuel for heating, the top gas still containing $CO_2$ is normally discharged out of the system.

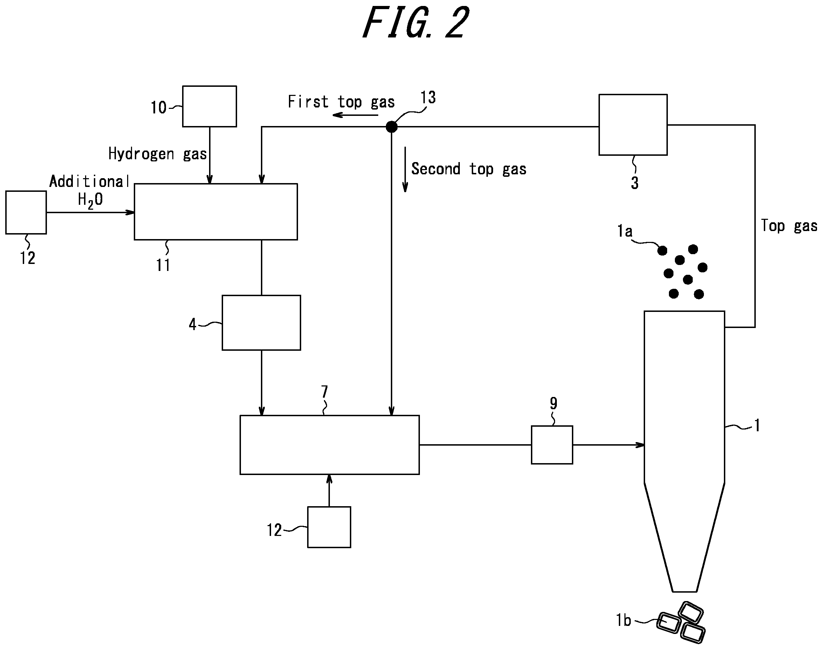

In contrast, in the method of producing reduced iron according to an embodiment of the present disclosure, as illustrated in FIG. 2 for example, the top gas discharged from the reduction furnace is distributed into first top gas and second top gas in a top gas distributor. Methane gas synthesized from the first top gas and hydrogen gas (hereinafter also referred to as regenerative methane gas) is used instead of hydrocarbon gas such as natural gas supplied from outside, as in the conventional process illustrated in FIG. 1. In the drawing, reference sign 10 is a hydrogen supply, 11 is a methane synthesizer, 12 is an $H_2O$ supply, and 13 is a top gas distributor. Here, the regenerative methane gas produced in the methane synthesizer 11 is supplied to the reformer 7 to be used as feed gas for the reducing gas.

The following is a description of each process of the method of producing reduced iron according to an embodiment of the present disclosure. The charging process, blowing process, and reduction process can be carried out in accordance with conventional methods, for example, in the same manner as in the conventional process described above, and therefore description is omitted here.

• Distribution Process

In the distribution process, for example, the top gas discharged from the reduction furnace is distributed into the first top gas and the second top gas in the top gas distributor. Distribution of the top gas and a flow rate controller are not particularly limited and may follow a conventional method. For example, a mass flow controller or the like may be used. A distribution ratio of the top gas is not particularly limited. For example, the distribution ratio of the top gas may be appropriately determined by a mass balance calculation according to the compositions of the reducing gas and the top gas. In addition, a portion of the top gas may be supplied to other loads depending on operating conditions and other factors, as long as the portion is 10 vol% or less of the top gas volume.

• Synthesis process

In the synthesis process, for example, regenerative methane gas is synthesized in a methane synthesizer from the first top gas distributed by the distribution process described above and hydrogen gas. $CH_4$ is synthesized, as mentioned above, from $CO_2$ and CO in the first top gas and $H_2$ according to the following methanation reaction equations in Expressions (i) and (ii).

$$CO_2 + 4H_2 \rightarrow CH_4 + 2H_2O \; \Delta H = \text{-165 kJ/mol} \ldots \qquad \text{(i)}$$

$$CO + 3H_2 \rightarrow CH_4 + H_2O \; \Delta H = \text{-206 kJ/mol} \ldots \qquad \text{(ii)}$$

For example, the first top gas and hydrogen gas supplied from outside the circulation system described above are introduced into the methane synthesizer. $CH_4$ is then synthesized in the methane synthesizer by the reactions in Expressions (i) and (ii). Conditions for $CH_4$ synthesis are not particularly limited, and a conventional method may be followed.

The compositions of the first top gas and the second top gas, described below, are basically the same as the composition of the top gas. The composition of the top gas can be exemplified as CO: 5 vol% to 50 vol%, $CO_2$: 5 vol% to 30 vol%, $H_2$: 5 vol% to 80 vol%, $H_2O$: 0 vol% to 35 vol%, with the balance being: 0 vol% to 20 vol%.

In addition to the first top gas, any other gas containing at least one of CO or $CO_2$ (hereinafter also referred to as "other gas") may be used in the synthesis process. Examples of the other gas include by-product gas of a steelmaking process, specifically blast furnace gas (BFG) and coke oven gas (COG).

Further, the source of hydrogen gas used in the synthesis process is not particularly limited and the hydrogen gas may be supplied and produced by any method. Methods of producing hydrogen gas include, for example, synthesis by electrolysis of water and by decomposition reactions of ammonia, hydrocarbons, and organic hydrides. However, when hydrocarbons or organic hydrides are used as a raw material, $CO_2$ is emitted in the hydrogen synthesis process. Therefore, from the viewpoint of further decreasing $CO_2$ emissions, synthesis by at least one of the electrolysis of water or decomposition of ammonia is preferred. Further, when hydrogen gas is produced by electrolysis of water, green hydrogen produced using electrical power from green energy sources such as solar, wind, and geothermal power may be used to decrease $CO_2$ emissions to zero. The $H_2$ concentration of the hydrogen gas is not particularly limited. The $H_2$ concentration of the hydrogen gas is preferably 90 vol% or more. The $H_2$ concentration of the hydrogen gas is more preferably 95 vol% or more. The $H_2$ concentration of the hydrogen gas may be 100 vol%.

Further, the amount of hydrogen gas supplied in the synthesis process (the amount of hydrogen gas supplied to the methane synthesizer) is preferably a stoichiometric amount relative to the amount of $CO_2$ and the amount of CO contained in the first top gas, based on the methanation reaction equations in Expressions (i) and (ii).

In the synthesis of $CH_4$, a typically used methanation catalyst may be used. Specifically, a transition metal catalyst such as Fe, Ni, Co, Ru, or the like may be used. Among these, Ni catalysts are the most active. Further, Ni catalysts also have high heat resistance and are usable up to about 500 °C. Therefore, Ni catalysts are particularly preferred. Further, iron ore may be used as a catalyst. Ores containing high crystallization water are particularly suitable for use as catalysts because the specific surface area increases when the water of crystallization is dehydrated.

The reactor of the methane synthesizer used in the synthesis process may be a fixed bed reactor, a fluidized bed reactor, an air flow bed reactor, or the like. Depending on the form of these reactors, the physical properties of the catalyst may be selected accordingly. Further, a heat exchanger may be disposed in the gas flow path downstream of the reactor to recover heat from the reaction heat (gas sensible heat) of the methanation reaction in each reactor. The recovered thermal energy can be returned upstream of the reactor, for example, and used to heat and maintain heat in the reactor. Further, the recovered thermal energy may be used for heating in other processes such as the reforming process.

Further, the methanation catalysts described above exhibit a stable high conversion rate during $CO_2$ methanation. However, during the methanation of CO, C is formed as an intermediate, as mentioned above. This causes C precipitation and catalyst poisoning. To prevent such catalyst poisoning, it is important to supply additional $H_2O$ to the synthesis process and to control the amount of the additional $H_2O$ according to the amount of CO and the amount of $H_2O$ contained in the first top gas. Details are as described under [Additional supply process] below.

Further, the temperature of the reactor of the methane synthesizer in which the methanation reaction occurs in the synthesis process is preferably controlled in the range from 200 °C to 550 °C. When the temperature of the reactor of the methane synthesizer is less than 200 °C, the reaction rate is slow and the methanation catalyst is not active enough. On the other hand, when the temperature of the reactor of the methane synthesizer exceeds 550 °C, the catalyst temperature may be excessively high and catalyst deactivation may be accelerated. Further, as indicated in Expressions (i) and (ii), the methanation reactions are exothermic reactions. Therefore, when the catalyst temperature is excessively high, the reaction equilibrium is biased to the left ($CO_2$ and CO side) of the reactions in Expressions (i) and (ii), resulting in lower methane yield. Therefore, the temperature of the reactor of the methane synthesizer is preferably controlled in the range from 200 °C to 550 °C.

In addition, the pressure in the reactor of the methane synthesizer, where the methanation reaction occurs during the synthesis process, is preferably controlled in a range from 3 atm to 10 atm. As indicated in Expressions (i) and (ii), the methanation reactions are molecular depletion reactions. Therefore, according to Le Chatelier's principle, the higher the pressure in the reactor of the methane synthesizer, the more easily the reaction equilibrium is biased to the right ($CH_4$) in the reactions of Expressions (i) and (ii), and therefore the higher the methane yield. However, when the pressure in the reactor of the methane synthesizer is excessively high, safety issues may arise. The pressure in the reactor of the methane synthesizer is therefore preferably controlled in the range from 3 atm to 10 atm.

When $H_2O$ produced as a byproduct of methane synthesis is introduced into the reformer, there may be an excess of $H_2O$ in the reforming process described below. Therefore, while considering the mass balance of the circulation system as a whole, it is preferable to dehydrate the regenerative methane gas using a dehydrator, as appropriate, prior to the reforming process described below.

Further, the $CH_4$ concentration of the regenerative methane gas is not particularly limited. In regenerative methane gas from which $H_2O$ is removed, that is, in regenerative methane gas after dehydration of the gas leaving the methane synthesizer by a dehydrator, the $CH_4$ concentration is preferably 80 vol% or more. The $CH_4$ concentration is more preferably 90 vol% or more. The $CH_4$ concentration in the regenerative methane gas may be 100 vol%.

• Additional supply process

The additional supply process is the process of supplying additional $H_2O$ to the synthesis process. Here, additional $H_2O$ is a term meaning the $H_2O$ supplied to the synthesis process separately from the first top gas.

In the additional supply process, it is important to control the amount of the additional $H_2O$ according to the

amount of CO and the amount of $H_2O$ contained in the first top gas.

As mentioned above, the top gas normally contains a certain amount of CO that was not used to reduce iron oxide in the reduction furnace, along with $CO_2$. Therefore, in the synthesis process, $CH_4$ is synthesized from CO in addition to $CO_2$. When $CH_4$ is synthesized from $CO_2$, $CH_4$ is synthesized according to the methanation reaction equation in Expression (i). When $CH_4$ is synthesized from CO, $CH_4$ is synthesized according to the methanation reaction equation in Expression (ii). However, when CO methanation reactions occur using typical methanation catalysts, catalyst poisoning occurs and the catalyst deactivates over a longer period of operation, resulting in lower methane yields.

Compounds with aldehyde (CHO) groups are formed as intermediates in the $CO_2$ methanation reaction. On the other hand, C is produced as an intermediate during the CO methanation reaction. This C precipitates on the catalyst and clogs the catalyst active sites, resulting in catalyst poisoning.

Here, when a sufficient amount of $H_2O$ is present in the synthesis process, C that is formed as intermediates during the CO methanation reaction and $H_2O$ react to form compounds with aldehyde (CHO) groups. This enables stable synthesis of $CH_4$ at a high yield by preventing catalyst poisoning in the synthesis process, even without separating $CO_2$ and CO contained in the top gas. As a result, it is possible to operate with high operational stability and achieve both further energy savings and a decrease in $CO_2$ emissions in the production of reduced iron.

Therefore, in the additional supply process, the amount of the additional $H_2O$ is controlled according to the amount of CO and the amount of $H_2O$ contained in the first top gas. In particular, the amount of the additional $H_2O$ is controlled so that the sum of the amount of $H_2O$ in the first top gas and the amount of the additional $H_2O$ supplied to the synthesis process is preferably 1.0 equivalent or more relative to the amount of CO in the first top gas. However, the $CO_2$ and CO methanation reactions in Expressions (i) and (ii) are equilibrium reactions, and an excessive increase in $H_2O$ will bias the equilibrium to the left ($CO_2$ and CO sides), resulting in a lower methane yield. Therefore, the amount of the additional $H_2O$ is controlled so that the sum of the amount of $H_2O$ in the first top gas and the amount of the additional $H_2O$ supplied to the synthesis process is preferably 3.0 equivalents or less, relative to the amount of CO in the first top gas. The sum is more preferably 2.0 equivalents or less.

Here, the equivalency is the molar equivalency. Further, the sum of the amount of $H_2O$ in the first top gas and the amount of the additional $H_2O$ supplied to the synthesis process being from 1.0 equivalent to 3.0 equivalents, relative to the amount of CO in the first top gas, can also be expressed as follows.

$$1.0 \leq ([\text{amount of } H_2O \text{ in first top gas (Nm}^3\text{/t)}] + [\text{amount of additional } H_2O \text{ supplied to synthesis process (Nm}^3\text{/t)}]) / [\text{amount of CO in first top gas (Nm}^3\text{/t)}] \leq 3.0$$

Hereinafter, ([amount of $H_2O$ in first top gas (Nm$^3$/t)] + [amount of additional $H_2O$ supplied to synthesis process (Nm$^3$/t)]) / [amount of CO in first top gas (Nm$^3$/t)] is also referred to as $H_2O$/CO.

That is, $H_2O$/CO is preferably 1.0 or more. Further, $H_2O$/CO is preferably 3.0 or less. $H_2O$/CO is more preferably 2.0 or less.

Further, the form of $H_2O$ supplied to the synthesis process in the additional supply process is preferably water vapor.

The $H_2O$ supply source used in the additional supply process is not particularly limited and supply may be by any method. For example, $H_2O$ supplied from outside the circulation system described above may be used, and $H_2O$ removed by the dehydrator described later may be reused.

Further, as described later, for example, when wet de-dusting using a scrubber or the like is carried out on the top gas, the top gas after de-dusting, and therefore the first top gas, also contains $H_2O$ equivalent to saturated water vapor content. The amount of $H_2O$ in the first top gas also contributes to the prevention of catalyst poisoning. Therefore, in addition to the amount of CO contained in the first top gas, the amount of $H_2O$ contained in the first top gas is also considered in control of the amount of the additional $H_2O$. The saturated water vapor content is a value determined by a temperature and a flow rate of the top gas. Further, the method of measuring the amount of $H_2O$ in the first top gas is not particularly limited, and may be measured using a dew point meter or moisture meter, for example.

• Reforming process

In the reforming process, the regenerative methane gas and the second top gas are used as feed gas to obtain reducing gas from the feed gas. For example, the regenerative methane gas and the second top gas are introduced into the reformer, and the regenerative methane gas and the second top gas are heated in the reformer. Then, in the reformer, the reforming reactions of the following Expressions (iii) and (iv) are used to produce reducing gas containing CO and $H_2$. By supplying water vapor to the reformer, the reforming reaction represented in Expression (iv) proceeds.

$$CH_4 + CO_2 \rightarrow 2CO + 2H_2 \quad \Delta H = 247 \text{ kJ/mol} \dots \qquad \text{(iii)}$$

$$CH_4 + H_2O \rightarrow CO + 3H_2 \ \Delta H = 206 \text{ kJ/mol} \ ... \qquad \text{(iv)}$$

The gas composition of the reducing gas is, for example, CO: 1 vol% to 60 vol%, $H_2$: 40 vol% to 99 vol%, with the balance being 0 vol% to 30 vol%.

As indicated by the positive enthalpies of formation in Expressions (iii) and (iv), both reactions in Expressions (iii) and (iv) are endothermic reactions. Therefore, by using green energy sources such as solar power, wind, geothermal energy, and the like as an energy source during the reforming reaction, $CO_2$ emissions may in principle be decreased to zero.

The reducing gas is then introduced into the reduction furnace through a blowing process. For example, the reducing gas is introduced into the reduction furnace using a reducing gas blowing device. The iron oxide is then reduced by the reducing gas in the reduction furnace to obtain reduced iron. On the other hand, the reducing gas after being used for iron oxide reduction is discharged from the reduction furnace as the top gas.

$CO_2/CO$, the ratio of the amount of $CO_2$ to the amount of CO contained in the top gas, is preferably 0.25 or more. $CO_2/CO$ is preferably 4.0 or less.

That is, the ratio of the amount of $CO_2$ to the sum of the amount of $CO_2$ and the amount of CO contained in the top gas, $CO_2/(CO_2 + CO)$, represents the reaction progress in the reduction reaction of iron oxide by CO in the following Expression (v). Further, $CO_2/(CO_2 + CO)$ depends on the equilibrium constant and reaction rate constant in Expression (v).

$$3CO + Fe_2O_3 \rightarrow 3CO_2 + 2Fe \ \Delta H = -247 \text{ kJ/kg-Fe} \ ... \qquad \text{(v)}$$

$$3H_2 + Fe_2O_3 \rightarrow 3H_2O + 2Fe \ \Delta H = 858 \text{ kJ/kg-Fe} \ ... \qquad \text{(vi)}$$

For example, when the grade of the raw material, iron ore, is decreased, the activation energy for reduction by $H_2$ increases. This decreases the reaction rate of Expression (vi), while increasing the reaction rate of Expression (v). As a result, the value of $CO_2/(CO_2 + CO)$ increases.

Further, the reduction by CO in Expression (v) is an exothermic reaction, whereas the reduction by $H_2$ in Expression (vi) is an endothermic reaction. Therefore, as the furnace temperature in the reduction furnace increases, the reaction rate in Expression (vi) increases while the reaction rate in Expression (v) decreases. As a result, the value of $CO_2/(CO_2 + CO)$ becomes smaller.

Here, from the viewpoint of stable operation, $CO_2/(CO_2 + CO)$ is preferably 0.2 or more. $CO_2/(CO_2 + CO)$ is more preferably 0.4 or more. Further, $CO_2/(CO_2 + CO)$ is preferably 0.8 or less. $CO_2/(CO_2 + CO)$ is more preferably 0.6 or less.

In detail:

$$0.2 \leq CO_2/(CO_2 + CO)$$

when transformed becomes

$$0.25 \leq CO_2/CO$$

$$CO_2/(CO_2 + CO) \leq 0.8$$

when transformed becomes

$$CO_2/CO \leq 4.0$$

Further,

$$0.4 \leq CO_2/(CO_2 + CO)$$

when transformed becomes

$$0.67 \leq CO_2/CO$$

Further,

$$CO_2/(CO_2 + CO) \leq 0.6$$

when transformed becomes

$$CO_2/CO \leq 1.5$$

In other words, $CO_2/CO$ is preferably 0.25 or more. $CO_2/CO$ is more preferably 0.67 or more. Further, $CO_2/CO$ is preferably 4.0 or less. $CO_2/CO$ is more preferably 1.5 or less.

The method of measuring $CO_2/CO$, which is the ratio of the amount of $CO_2$ to the amount of CO contained in the top gas, is not particularly limited, but may be measured, for example, as follows. In other words, the flow rate of the top gas (converted into standard condition) may be measured using a flow meter at any point in the top gas distributor. Further, the top gas may be collected from a sampling port and the volume concentration of $CO_2$ and CO of the gas may be measured by gas chromatography. $CO_2/CO$ is then determined by dividing the volume concentration of $CO_2$ in the gas by the volume concentration of CO in the gas. The flow rate of the top gas (converted into standard condition) [$Nm^3/h$] may also be converted to the flow rate (converted into standard condition) per tonne of reduced iron (DRI) produced [$Nm^3/t$]. Further, the flow rate of other gas may be measured in the same manner.

Further, dust in the first top gas introduced into the synthesis process may cause clogging and other problems. Therefore, it is preferable to carry out de-dusting of the top gas or the first top gas prior to the synthesis process. In particular, it is preferable to carry out de-dusting of the top gas prior to the distribution process. Any deduster may be used for de-dusting. For example, in a blast furnace, dry dust removal is carried out on the top gas using a cyclone or the like, followed by wet dust removal using a scrubber or the like. In wet dust removal, water is used to wash away dust, which simultaneously cools the top gas. As a result, most of the water vapor in the top gas condenses and is collected with wastewater. However, in this case, $H_2O$ equivalent to the saturated water vapor content is present in the top gas, depending on the temperature of the top gas after cooling. As mentioned above, the amount of $H_2O$ in the first top gas also contributes to the prevention of catalyst poisoning. Therefore, it is preferable to carry out de-dusting by wet dust removal, for example, so that $H_2O$ equivalent to the saturated water vapor content is present in the top gas.

Further, in the method of producing reduced iron according to an embodiment of the present disclosure, the flow rate of the reducing gas supplied to the reduction furnace is preferably 1500 $Nm^3/t$ or more. The flow rate is preferably 3500 $Nm^3/t$ or less. That is, when the flow rate of the reducing gas supplied to the reduction furnace is too low, production decreases and product properties degrade. On the other hand, when the flow rate of the reducing gas supplied to the reduction furnace is excessive, gas flow resistance in the reduction furnace increases and the raw material (pellet) in the reduction furnace does not descend. Therefore, the flow rate of the reducing gas supplied to the reduction furnace is preferably 1500 $Nm^3/t$ or more. The flow rate is preferably 3500 $Nm^3/t$ or less.

Further, the iron oxide raw material used in the method of producing reduced iron according to an embodiment of the present disclosure is, for example, iron ore. Examples include lumped iron ore (lump ore), pellets (powdered iron ore hardened into a spherical shape), and the like. The grade of iron ore used as iron oxide raw material, that is, the iron content, is not particularly limited. From the perspective of reduction in a shaft furnace, the iron content is typically preferably 65 mass% or more. However, in recent years, prices are expected to soar due to the depletion of high-grade iron ore from South America and other sources. Therefore, low-grade iron ore that has an iron content of 63 mass% or less is preferably used as required. Such low-grade iron ore is inexpensive and abundant, and places of origin include, for example, Australia.

In addition, the method of producing reduced iron according to an embodiment of the present disclosure describes, among other things, the use of a shaft furnace as a direct reduction iron-making process. However, the type of reduction furnace is not limited to this, and may be a fluidized bed, a rotary kiln, a rotary hearth furnace (RHF), or the like. Shaft furnaces are preferred as reduction furnaces because of their high production efficiency, ratio of utilization and operational stability. Further, the majority of direct reduction furnaces in operation worldwide are Midrex® (Midrex is a registered trademark in Japan, other countries, or both) and Hyl® (Hyl is a registered trademark in Japan, other countries, or both), which are shaft furnace systems.

EXAMPLES

Examples of the present disclosure are described below.

(Examples 1)

In the circulation system illustrated in FIG. 2, reduced iron was produced under the following conditions. The raw material used was iron ore pellets from Brazil.

Flow rate of the reducing gas supplied to the reduction furnace: 2200 $Nm^3/t$
$H_2/CO$ of the reducing gas supplied to the reduction furnace: 2.0
Operation period: 28 days
Production rate of reduced iron: 120 t/h
Gas composition (volume ratio) in the top gas during steady-state operation:
$H_2$ : CO : $CO_2$ : $H_2O$ = 48 : 30 : 9 : 13

In the Example, the amounts of $CO_2$, CO, and $H_2O$ contained in the top gas were measured, and the amount of the additional $H_2O$ supplied to the synthesis process was controlled according to the amount of CO and the amount of $H_2O$ contained in the first top gas so that $H_2O/CO$ was 1.0.

On the other hand, for comparison, as Comparative Example 1, the production of reduced iron was carried out without supplying the additional $H_2O$ to the synthesis process.

Further, as Comparative Example 2, reduced iron was produced by supplying a constant amount of the additional $H_2O$ to the synthesis process without controlling the amount of the additional $H_2O$ according to the amount of CO and the amount of $H_2O$ contained in the first top gas.

For both Comparative Examples 1 and 2, conditions other than those described above were the same as those for the Example.

As a result, the Example was able to operate stably with a high methane yield and prevention of catalyst poisoning over the entire 28-day operation period under a healthy mass balance in the circulation system illustrated in FIG. 2, that is, in a system where the top gas is circulated and reused, which is extremely advantageous in realizing energy savings. Further, $CO_2$ emissions from the circulation system could be decreased to zero.

In contrast, for Comparative Example 1, catalyst poisoning by CO methanation reaction occurred partway through the operation period, and stable operation at a high methane yield was no longer possible, so the operation was unavoidably suspended. The locations where catalyst poisoning occurred were confirmed by the procedure described below, and it was found that catalyst poisoning had occurred at three or more locations.

For Comparative Example 2, stable operation under a healthy mass balance became impossible partway through the operation period, so the operation was unavoidably suspended. The methane yield (from the start of operation to suspension), as evaluated in the manner described below, was less than 93 %.

(Examples 2)

Except for the conditions listed in Table 1, the production of reduced iron was carried out under the same conditions as in the Example of Examples 1, except that $H_2O/CO$ was changed in various ways.

Under each set of conditions, the methane yield was measured. Specifically, the methane yield was measured hourly over the entire operation period (28 days) and an average of the measured methane yields was determined. The methane yield was calculated by the following expression.

[methane yield (%)] = (1 - [amount of $CO_2$ and CO contained in regenerative methane gas discharged after synthesis of $CH_4$ in synthesis process ($Nm^3/t$)] ÷ [amount of $CO_2$ and CO introduced in synthesis process ($Nm^3/t$)]) $\times$ 100

The methane yield was then evaluated according to the following criteria. The evaluation results are listed in Table 1.

1 (pass, particularly outstanding): methane yield of 97 % or more
2 (pass, outstanding): methane yield from 95 % to less than 97 %
3 (pass): methane yield from 93 % to less than 95 %
4 (fail): methane yield less than 93 %.

After the 28-day operation period, the methanation catalyst was evaluated for resistance to catalyst poisoning (hereinafter also referred to as catalyst poisoning resistance) in the following manner.

After the 28-day operation period, the entire surface of the methanation catalyst was observed visually and with an optical microscope to check for the presence of discolored locations. When a discolored location was identified, the presence or absence of a fibrous material formation was checked by SEM and the presence or absence of a C intensity increase was checked by SEM-EDX analysis to determine whether or not catalyst poisoning had occurred in the discolored location. Fibrous material formation on the surface of the methanation catalyst and an increase in intensity of C are phenomena caused by catalyst poisoning, and when at least one of these phenomena was observed in a discolored location, it was judged that catalyst poisoning had occurred. The locations of catalyst poisoning on the surface of the methanation catalyst were counted and evaluated for catalyst poisoning resistance according to the following criteria. The evaluation results are listed in Table 1.

1 (pass, particularly outstanding): no locations where catalyst poisoning occurred
2 (pass): one or two locations where catalyst poisoning occurred
3 (fail): three or more locations where catalyst poisoning occurred

[Table 1]

Table 1

| No. | First top gas [$Nm^3/t$] | | | Amount of additional $H_2O$ [$Nm^3/t$] | $H_2O/CO$ | Evaluation result | |
|---|---|---|---|---|---|---|---|
| | $CO_2$ | CO | $H_2O$ | | | Methane yield | Catalyst poisoning resistance |
| 1 | 308 | 440 | 180 | 172 | 0.8 | 1 | 2 |
| 2 | 308 | 396 | 228 | 168 | 1.0 | 1 | 1 |
| 3 | 352 | 462 | 442 | 159 | 1.3 | 1 | 1 |
| 4 | 374 | 550 | 539 | 451 | 1.8 | 1 | 1 |
| 5 | 462 | 528 | 418 | 638 | 2.0 | 1 | 1 |
| 6 | 330 | 506 | 275 | 990 | 2.5 | 2 | 1 |
| 7 | 374 | 517 | 613 | 1455 | 4.0 | 3 | 1 |

As indicated in Table 1, in all cases from No. 1 to No. 7, where the amount of the additional $H_2O$ supplied to the synthesis process was controlled according to the amount of CO and $H_2O$ contained in the first top gas, stable operation with a high methane yield and prevention of catalyst poisoning was possible over the entire operation period of 28 days under a healthy mass balance. That is, stable operation was achieved in the circulation system illustrated in FIG. 2, where the top gas is circulated and reused, which is extremely advantageous in realizing energy savings. Further, $CO_2$ emissions from the circulation system could be decreased to zero.

Among these, in No. 2 to No. 5 in particular, the amount of the additional $H_2O$ supplied to the synthesis process was controlled so that $H_2O/CO$ was from 1.0 to 2.0, that is, the sum of the amount of $H_2O$ contained in the first top gas and the amount of the additional $H_2O$ supplied to the synthesis process was between 1.0 equivalent and 2.0 equivalents relative to the amount of CO contained in the first top gas, and particularly high methane yields and particularly outstanding catalyst poisoning resistance were obtained.

REFERENCE SIGNS LIST

1       reduction furnace
1a      iron oxide
1b      reduced iron
3       deduster
4       dehydrator
5       natural gas supply
6       air supply
7       reformer
9       reducing gas blowing device
10      hydrogen supply

11    methane synthesizer
12    $H_2O$ supply
13    top gas distributor

**Claims**

1.  A method of producing reduced iron, the method comprising:

    a charging process of charging iron oxide into a reduction furnace;
    a blowing process of blowing reducing gas into the reduction furnace;
    a reduction process, in the reduction furnace, of reducing the iron oxide by the reducing gas to obtain reduced iron;
    a distribution process of distributing top gas discharged from the reduction furnace into a first top gas and a second top gas;
    a synthesis process of synthesizing regenerative methane gas from the first top gas and hydrogen gas; and
    an additional supply process of supplying additional $H_2O$ to the synthesis process; and
    a reforming process of using the regenerative methane gas and the second top gas as feed gas to obtain the reducing gas from the feed gas, wherein
    an amount of the additional $H_2O$ supplied to the synthesis process is controlled according to an amount of CO and an amount of $H_2O$ contained in the first top gas.

2.  The method of producing reduced iron according to claim 1, wherein the amount of the additional $H_2O$ supplied to the synthesis process is controlled so that the sum of the amount of $H_2O$ contained in the first top gas and the amount of the additional $H_2O$ supplied to the synthesis process is from 1.0 equivalent to 3.0 equivalents relative to the amount of CO contained in the first top gas.

3.  The method of producing reduced iron according to claim 1, wherein the amount of the additional $H_2O$ supplied to the synthesis process is controlled so that the sum of the amount of $H_2O$ contained in the first top gas and the amount of the additional $H_2O$ supplied to the synthesis process is from 1.0 equivalent to 2.0 equivalents relative to the amount of CO contained in the first top gas.

4.  The method of producing reduced iron according to any one of claims 1 to 3, wherein $CO_2/CO$, a ratio of an amount of $CO_2$ to the amount of CO contained in the top gas, is from 0.25 to 4.0.

5.  The method of producing reduced iron according to any one of claims 1 to 3, wherein de-dusting of the top gas or the first top gas is carried out prior to the synthesis process.

6.  The method of producing reduced iron according to claim 4, wherein de-dusting of the top gas or the first top gas is carried out prior to the synthesis process.

7.  The method of producing reduced iron according to any one of claims 1 to 3, wherein dehydration of the regenerative methane gas is carried out prior to the reforming process.

8.  The method of producing reduced iron according to claim 4, wherein dehydration of the regenerative methane gas is carried out prior to the reforming process.

9.  The method of producing reduced iron according to claim 5, wherein dehydration of the regenerative methane gas is carried out prior to the reforming process.

10. The method of producing reduced iron according to claim 6, wherein dehydration of the regenerative methane gas is carried out prior to the reforming process.

# FIG. 1

EP 4 596 721 A1

*FIG. 2*

Hydrogen gas

Additional H₂O

First top gas  13

Second top gas

Top gas

EP 4 596 721 A1

## INTERNATIONAL SEARCH REPORT

| International application No. |
|---|
| **PCT/JP2023/045558** |

### A. CLASSIFICATION OF SUBJECT MATTER

***C21B 5/06***(2006.01)i; ***C07C 1/04***(2006.01)i; ***C07C 1/12***(2006.01)i; ***C10K 3/06***(2006.01)i; ***C21B 5/00***(2006.01)i; ***C21B 13/02***(2006.01)i; ***F27D 17/00***(2006.01)i

FI: C21B5/06; C07C1/04; C07C1/12; C10K3/06; C21B5/00 321; C21B13/02; F27D17/00 104G

According to International Patent Classification (IPC) or to both national classification and IPC

### B. FIELDS SEARCHED

Minimum documentation searched (classification system followed by classification symbols)

C21B5/06; C07C1/04; C07C1/12; C10K3/06; C21B5/00; C21B13/02; F27D17/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2014-028984 A (JFE STEEL CORPORATION) 13 February 2014 (2014-02-13) claim 1, fig. 1 | 1-10 |
| A | JP 2009-120897 A (JFE STEEL CORPORATION) 04 June 2009 (2009-06-04) paragraphs [0025]-[0026] | 1-10 |

☐ Further documents are listed in the continuation of Box C.    ☑ See patent family annex.

| | |
|---|---|
| \*   Special categories of cited documents: | "T"   later document published after the international filing date or priority date and not in conflict with the application but cited to understand the principle or theory underlying the invention |
| "A"   document defining the general state of the art which is not considered to be of particular relevance | |
| "D"   document cited by the applicant in the international application | "X"   document of particular relevance; the claimed invention cannot be considered novel or cannot be considered to involve an inventive step when the document is taken alone |
| "E"   earlier application or patent but published on or after the international filing date | |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| **13 March 2024** | **26 March 2024** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**INTERNATIONAL SEARCH REPORT**
Information on patent family members

International application No.

**PCT/JP2023/045558**

| Patent document cited in search report | | | Publication date (day/month/year) | Patent family member(s) | Publication date (day/month/year) |
|---|---|---|---|---|---|
| JP | 2014-028984 | A | 13 February 2014 | (Family: none) | |
| JP | 2009-120897 | A | 04 June 2009 | (Family: none) | |

Form PCT/ISA/210 (patent family annex) (July 2022)

**EP 4 596 721 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017088912 A
- JP 6190522 B